# FASCICULE DE BREVET EUROPEEN

(11) **EP 0 769 972 B1**
(45) Date de publication et mention de la délivrance du brevet: **09.08.2000**
(21) Numéro de dépôt: 95939320.8
(22) Date de dépôt: 03.11.1995
(51) Int. Cl.: A61M 3/02

(54) **INSTRUMENT POUR NETTOYER LE CONDUIT AUDITIF EXTERNE**
VORRICHTUNG ZUR REINIGUNG DES ÄUSSEREN GEHÖRGANGES
DEVICE FOR CLEANING THE EXTERNAL AUDITORY MEATUS

(30) Priorité: 04.11.1994 FR 9413497
(43) Date de publication de la demande: 02.05.1997
(73) Titulaire: Baguant, Kamalkishore, 59620 Aulnoye-Aymeris (FR)
(72) Inventeur: Baguant, Kamalkishore, 59620 Aulnoye-Aymeris (FR)
(74) Mandataire: Hénnion, Jean-Claude
(86) Numéro de dépôt international: FR9501448
(87) Numéro de publication internationale: WO9614098

(56) Documents cités:
- DE-C- 88 135
- FR-A- 391 130
- FR-A- 1 110 485
- GB-A- 2 123 697
- US-A- 1 925 230
- US-A- 3 054 403
- US-A- 3 921 635
- US-A- 4 206 756
- PATENT ABSTRACTS OF JAPAN vol. 017 no. 622 (C-1130) ,17 Novembre 1993 & JP,A,05 192394 (YASUO SOTOMA) 3 Août 1993,

## Description

La présente invention a pour principal objet un embout de pulvérisation d'un liquide à l'intérieur du conduit auditif externe d'une oreille. Elle trouve plus particulièrement son application dans le nettoyage du conduit auditif externe, par pulvérisation d'un soluté physiologique d'eau de mer, afin d'éliminer le cérumen de la paroi interne du conduit auditif.

On a déjà proposé dans le brevet US 4 206 756 un embout permettant de pulvériser un liquide de lavage à l'intérieur du conduit auditif externe d'une oreille, en vue de le débarrasser du cérumen, qui est secrété par les glandes sébacées tapissant la paroi interne de ce conduit auditif. Cet embout de pulvérisation se présente sous la forme d'un tube qui est dimensionné pour être introduit dans le conduit auditif externe, de telle sorte que son extrémité de pulvérisation vienne à proximité de la membrane tympanique. Cette extrémité de pulvérisation est fermée au niveau de l'axe du tube, et est percée de plusieurs orifices, qui sont régulièrement répartis sur toute la périphérie du tube. Le liquide de lavage est introduit sous pression à l'intérieur du tube, et est évacué par les orifices de l'extrémité de pulvérisation, sous la forme d'une pluralité de jets de pulvérisation qui sont orientés dans une direction oblique par rapport à l'axe du tube. Plus particulièrement ces jets de pulvérisation font sensiblement un angle d'environ 45° par rapport à l'axe du tube. Il en résulte que les jets de pulvérisation ne viennent pas directement au contact de la membrane tympanique, mais sont projetés contre la paroi interne du conduit auditif. Cette orientation des jets de pulvérisation est avantageuse, car elle évite les risques de détérioration, sous l'impact des jets de pulvérisation, de la membrane tympanique, qui est un organe très fragile.

Cependant l'embout de pulvérisation décrit dans le brevet US 4 206 756 présente les deux inconvénients majeurs suivants. Avec ce type d'embout de pulvérisation, l'air initialement présent dans le conduit auditif, entre l'extrémité de l'embout de pulvérisation et la membrane tympanique, une fois l'embout introduit dans le conduit auditif, peut uniquement s'évacuer entre la paroi interne du conduit auditif et la périphérie externe de l'embout de pulvérisation. Par conséquent, s'il arrive que l'espace annulaire entre la paroi interne du conduit auditif et la périphérie externe de l'embout est obstruée partiellement ou totalement, l'injection du liquide de lavage occasionne une surpression dangereuse au niveau de la membrane tympanique, l'air ne pouvant plus être évacué correctement. Il peut dans ce cas en résulter une destruction irréversible de la membrane tympanique.

Les causes d'obstruction de l'espace annulaire permettant l'évacuation de l'air peuvent avoir deux origines. La première origine peut consister dans un dimensionnement trop important de l'embout, par rapport à l'entrée du conduit auditif constituée par le méat auditif. Il faut ici souligner que le méat auditif n'est pas parfaitement rectiligne, et est constituée d'une paroi cartilagineuse déformable. Par conséquent, pour introduire l'embout de pulvérisation du brevet US 4 206 756, on est amené à déformer cette paroi cartilagineuse. Il y a donc un risque que l'embout de pulvérisation, bien que présentant une section suffisamment faible pour pouvoir être introduit dans le conduit auditif, occasionne lors de son introduction dans le conduit auditif une déformation du méat auditif, qui est telle que la paroi cartilagineuse du méat auditif se referme sur la périphérie externe de l'embout, ce qui occasionne une obstruction de l'espace annulaire entre l'embout de pulvérisation et la paroi interne du conduit auditif. La seconde origine du risque d'obstruction provient de l'accumulation possible de cérumen entre l'embout de pulvérisation et la paroi interne du conduit auditif.

Le second inconvénient de l'embout de pulvérisation du brevet US 4 206 756, réside dans le fait que rien n'empêche un utilisateur d'introduire trop profondément cet embout dans le conduit auditif externe et par là même de venir perforer la membrane tympanique. Il faut ici souligner qu'il n'est pas envisageable avec un tel embout de pulvérisation de concevoir une portion de plus grand diamètre, destinée à venir en butée contre l'oreille , afin d'éviter une introduction trop profonde de l'embout de pulvérisation, car une telle portion occasionnerait une obstruction de l'espace annulaire, faisant obstacle à l'échappement de l'air.

Le but de la présente invention est de proposer un embout de pulvérisation d'un liquide à l'intérieur du conduit auditif externe d'une oreille, qui permet de pallier les inconvénients constatés de l'embout de pulvérisation précité, qui sont liés aux risques d'obstruction de l'espace annulaire d'évacuation de l'air entre la paroi interne du conduit auditif et la partie de l'embout de pulvérisation introduite dans le conduit.

De manière connue, notamment par le brevet US 4 206 756, l'embout de l'invention comporte une tête de pulvérisation oblongue, percée d'au moins un orifice d'évacuation, qui communique avec un canal d'amenée du liquide, et dont l'axe de sortie diverge par rapport à l'axe longitudinal de la tête de pulvérisation en étant orienté en direction de l'extrémité de ladite tête, de telle sorte qu'une fois la tête de pulvérisation introduite à l'intérieur du conduit auditif, le liquide pulvérisé par l'orifice d'évacuation se trouve projeté directement contre la paroi interne du conduit auditif.

De manière caractéristique selon l'invention, l'embout comprend une cavité intérieure qui est distincte du canal d'amenée du liquide et qui présente au moins une première ouverture au niveau de la tête de pulvérisation et une deuxième ouverture au niveau de la partie de l'embout de pulvérisation opposée à la tête de pulvérisation, de telle sorte qu'une fois la tête de l'embout de pulvérisation introduite dans le conduit auditif, la partie de l'embout pourvue de la deuxième ouverture est située à l'extérieur de l'oreille, et l'air initialement présent entre l'extrémité de la tête de l'embout de pulvérisation et la membrane tympanique peut s'évacuer librement vers l'extérieur de l'oreille par la cavité intérieure.

Le canal d'amenée d'un liquide jusqu'à un orifice d'évacuation pourra par exemple consister en un tuyau flexible relié audit orifice et logé à l'intérieur de la cavité intérieure, ou encore en une canalisation intégrée dans la paroi de l'embout. De préférence, l'embout de pulvérisation comporte deux éléments concentriques, l'élément intérieur délimitant la cavité intérieure, et l'élément extérieur délimitant avec l'élément intérieur un espace annulaire. Dans ce cas, le ou les orifices d'évacuation sont pratiqués dans l'élément extérieur et communiquent avec l'espace annulaire, qui fait office de canal d'amenée du liquide.

De préférence, la partie de l'embout de pulvérisation comportant la deuxième ouverture présente une dimension suffisamment importante pour empêcher son passage dans le méat auditif.

Avantageusement les première et deuxième ouvertures seront alignées l'une par rapport à l'autre dans l'axe longitudinal de l'embout, en sorte de permettre l'inspection visuelle du conduit auditif externe.

Plus particulièrement, la composante longitudinale de l'axe de sortie de chaque orifice d'évacuation fait, avec l'axe longitudinal de la tête de pulvérisation, un angle α strictement compris entre 30° et 90°, et de préférence égal à 60°. La valeur minimale de 30° pour l'angle α permet en pratique de pulvériser des jets de liquide dans une direction suffisamment divergente par rapport à la membrane tympanique pour garantir que ces jets ne viendront pas directement au contact de la membrane tympanique La valeur moyenne préferée de 60° de l'angle α permet en pratique d'obtenir un angle d'incidence par rapport a la paroi interne du conduit auditif, qui occasionne un écoulement optimal ultérieur des jets de liquide à la surface de la paroi interne du conduit auditif, en direction de la membrane tympanique.

De préférence, la composante transversale de l'axe de sortie de chaque orifice d'évacuation fait, avec l'axe longitudinal de la tête de pulvérisation, un angle β strictement compris entre 0 et 90°, et de préférence égal à 45°. Cet angle β combiné avec l'angle α permet de conférer au jet de liquide un trajet hélicoïdal à la surface de la paroi interne du conduit auditif externe, en direction de la membrane tympanique, ce qui permet d'augmenter la surface de contact du liquide avec la paroi interne.

Dans une variante préférée de réalisation, l'embout de pulvérisation comprend au moins trois orifices d'évacuation, qui sont régulièrement répartis sur toute la périphérie de la tête de pulvérisation, et dont les angles β sont identiques, et de préférence égaux à 45°. Dans cette variante de réalisation, les jets de liquide pulvérisé ont tendance à se propager à la surface de la paroi interne du conduit auditif selon au moins trois trajets hélicoïdaux complémentaires.

La présente invention a également pour autre objet un dispositif de pulvérisation équipé d'un embout pour la pulvérisation d'un liquide à l'intérieur du conduit auditif externe.

Selon une première variante, le dispositif comporte un récipient qui est rempli avec un liquide sous pression, qui est fermé par un clapet anti-retour, et sur lequel est monté un embout de pulvérisation de l'invention, dont le ou les canaux d'amenée du liquide communiquent avec le récipient, et qui permet d'actionner le clapet anti-retour pour libérer le liquide contenu dans le récipient à destination du ou des orifices d'évacuation de l'embout.

Selon une deuxième variante de réalisation, le dispositif comporte un embout de pulvérisation de l'invention, dont le ou les canaux d'amenée du liquide communiquent avec un récipient ouvert, par l'intermédiaire d'un conduit unique ; ce conduit est équipé d'une poire permettant le pompage manuel d'un liquide contenu dans le récipient.

Dans les deux variantes, le liquide sera avantageusement un soluté stérile physiologique d'eau de mer.

D'autres caractéristiques et avantages de l'invention ressortiront de la description suivante de plusieurs modes particuliers de réalisation d'un embout de pulvérisation de l'invention, et de deux variantes de réalisation d'un dispositif de pulvérisation, laquelle description est donnée à titre d'exemple non limitatif et en référence au dessin annexé sur lequel :
- La figure 1 est une vue de face d'un embout de pulvérisation de l'invention, faisant également office de spéculum,
- La figure 2 est une vue en coupe de l'embout de pulvérisation de la figure 1, introduit à l'intérieur du conduit auditif externe,
- la figure 3 est vue en coupe d'une deuxième variante de réalisation d'un embout de pulvérisation comportant un espace annulaire faisant office de canal d'amenée du liquide,
- Les figures 4 et 5 sont des représentations schématiques de dispositifs de pulvérisation réalisés respectivement selon les première et deuxième variantes de l'invention.

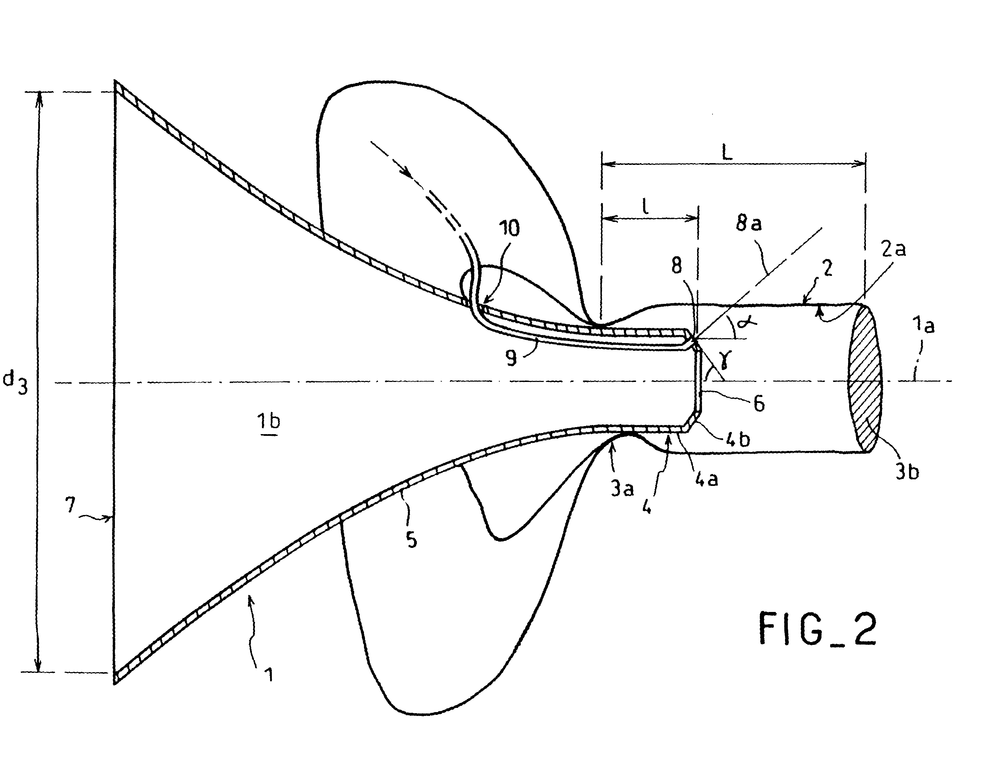

On a représenté à la figure 2 un exemple particulier de réalisation d'un embout de pulvérisation 1 de l'invention, introduit dans le conduit auditif externe 2 d'une oreille. Ce conduit auditif externe, représenté très schématiquement, communique avec l'extérieur par l'intermédiaire du méat auditif 3a, et est fermé à son extrémité opposée par la membrane tympanique 3b.

L'embout de pulvérisation 1 a la forme générale d'un spéculum, couramment utilisé par les spécialistes O.R.L., pour réaliser l'inspection visuelle du conduit auditif externe et de la membrane tympanique Cet embout a donc la forme d'un cornet creux, d'axe 1a, et présente une cavité intérieure 1b. Il comprend avec une première partie 4 de forme tubulaire, qui est dimensionnée pour être introduite dans le conduit auditif externe 2 par l'intermédiaire du méat auditif 3a, et qui se prolonge par une deuxième partie 5 évasée, destinée à rester à l'extérieur du conduit auditif externe. La cavité intérieure 1b est pourvue de deux ouvertures 6 et 7 qui sont pratiquées respectivement au niveau des faces d'extrémité des parties 4 et 5, et qui sont alignées l'une par rapport à l'autre dans l'axe 1a de l'embout 1, en sorte de permettre l'inspection visuelle du conduit auditif externe 2.

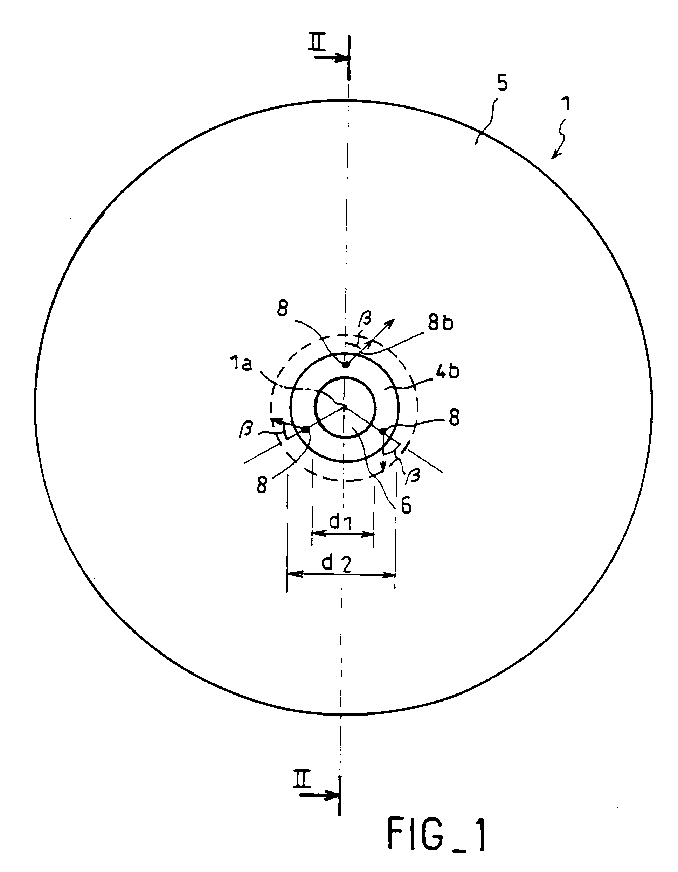

La partie 4 précitée correspond à la tête de pulvérisation de l'embout 1. Elle comporte plus particulièrement une première partie cylindrique 4a, qui est centrée sur l'axe 1a, et qui se prolonge par une partie tronconique 4b, d'angle γ, jusqu'à l'ouverture 6. La partie tronconique 4b est percée de trois orifices d'évacuation 8 qui sont régulièrement répartis sur toute sa périphérie (figure 1), un seul de ces orifices étant visible sur la figure 2. Chaque orifice d'évacuation 8 communique avec un canal 9 qui est interne à l'embout 1, et qui débouche à l'extérieur dans la partie 5 de l'embout 1 par l'intermédiaire d'un orifice 10. Chaque canal 9 a pour fonction d'amener un liquide jusqu'à l'orifice d'évacuation 8 correspondant. Les canaux 9 peuvent être par exemple de fins tuyaux flexibles, reliés entre eux à l'extérieur de la cavité intérieure de l'embout 1. Il pourrait également s'agir de canaux intégrés directement dans la paroi de l'embout de pulvérisation.

Si l'on se réfère à la figure 2, la partie 5 évasée de l'embout de pulvérisation présente une dimension suffisamment importante par rapport au méat auditif 3a pour ne pas pouvoir être introduite dans le conduit auditif 2. Par conséquent, si l'on cherche à introduire la tête de pulvérisation plus profondément à l'intérieur du conduit auditif 2, la partie évasée 5 vient en butée contre l'oreille, et empêche que l'embout ne soit introduit trop profondément. De plus, la deuxième ouverture 7 reste toujours nécessairement située à l'extérieur de l'oreille. Il revient à l'homme du métier de prévoir une longueur l de la tête de pulvérisation , qui soit inférieure à la longueur L du conduit auditif 2, de telle sorte qu'une fois la tête de pulvérisation 4 introduite le plus profondément possible dans le conduit auditif 2, la partie évasée 5 venant en butée contre l'oreille, l'extrémité de pulvérisation de la partie 4 ne vienne pas en contact avec la membrane tympanique 3b. Dans un exemple précis de réalisation, la longueur l de la tête de pulvérisation 4 valait 15mm ; le diamètre d₁ de l'ouverture 6 et le plus grand diamètre d₂ de la partie tronconique 4b valaient respectivement 3mm et 5mm ; le diamètre d₃ de l'ouverture 7 valait environ 30mm.

Conformément à l'invention, lorsque l'on injecte un liquide sous pression dans les canaux 9, ce liquide est évacué par les orifices 8 sous la forme de trois jets projetés directement contre la paroi interne 2a du conduit auditif 2. L'air qui était initialement présent entre la membrane tympanique 3b et la tête de pulvérisation 4 de l'embout, peut librement pénétrer dans la cavité intérieure 1b par la première ouverture 6 et être évacué vers l'extérieur de l'oreille par la deuxième ouverture 7. En outre, le liquide qui est injecté dans le conduit auditif externe 2, et qui permet de le débarrasser de son cérumen, peut avantageusement être évacué par l'ouverture 6 et s'écouler à l'intérieur de l'embout 1 jusqu'à l'ouverture 7 en vue d'être recueilli. Il convient de noter que le méat auditif 3a forme un goulot d'étranglement par rapport au conduit auditif externe 2. Ce méat auditif étant réalisé en cartilage, il peut être facilement déformé lors de l'introduction de la partie 4 dans le conduit auditif externe 2, et se referme sur l'embout de pulvérisation, tel que cela est illustré sur la figure 2. L'air présent dans le conduit auditif 2 peut très difficilement s'évacuer, voir même ne pas du tout s'évacuer, entre la paroi interne 2a du conduit auditif 2 et la paroi externe de la tête de pulvérisation 4. Par conséquent, la cavité intérieure 1b de l'embout qui a pour principale fonction de faire communiquer l'intérieur du conduit auditif 2 avec l'extérieur de l'oreille par l'intermédiaire des deux ouvertures 6 et 7, permet avantageusement d'éviter tout risque de surpression au niveau de la membrane tympanique 3b, lors de l'injection d'un liquide sous pression.

On a représenté sur les figures 1 et 2 les composantes respectivement transversale 8b et longitudinale 8a des axes de sortie des orifices d'évacuation 8 ; chaque axe de sortie d'un orifice d'évacuation correspond en pratique à la direction prise par le jet de liquide qui est pulvérisé par l'ouverture d'évacuation correspondante. Si l'on se réfère à la figure 2, la composante longitudinale 8a de l'axe de sortie d'un orifice d'évacuation 8 fait un angle droit avec la surface de la partie tronconique 4b. Il en résulte que cette composante longitudinale 8a fait, par rapport à l'axe 1a, un angle α dont la mesure est sensiblement égale à celle de l'angle γ de la partie tronconique 4b. Cet angle α correspond sensiblement à l'angle d'incidence d'un jet de liquide par rapport à la paroi interne du conduit auditif externe 2. Conformément à l'invention la mesure de cet angle α est strictement comprise entre 30° et 90°. En effet, si cet angle α était inférieur à 30°, la pulvérisation du liquide se ferait directement en direction de la membrane tympanique 3b, et exercerait une pression traumatisante pour cette membrane. Par ailleurs, si la mesure de l'angle α valait 90°, on éviterait certes tout risque de détérioration de la membrane tympanique, mais le liquide pulvérisé resterait au niveau de l'orifice d'évacuation, et ne pourrait pas se propager à la surface de la paroi interne du conduit auditif externe en direction de la membrane tympanique. La mesure de l'angle α doit donc être judicieusement choisie entre ces deux valeurs de 30° et 90° en étant suffisamment faible pour permettre une progression optimale du liquide dans le conduit auditif externe, en direction de la membrane tympanique, mais suffisamment important pour que le liquide ne soit pas projeté directement au contact de cette membrane et exerce une pression traumatisante sur celle-ci. En pratique, la mesure de l'angle α sera de préférence égale à 60°.

Si l'on se réfère à la figure 1, la composante transversale 8b de chaque orifice d'évacuation 8 ne se situe pas dans le plan médian passant par l'axe 1a, mais forme un angle β par rapport à ce plan. Dans l'exemple particulier illustré, la mesure des angles β est identique pour chaque orifice d'évacuation et est sensiblement égale à 45°. Grâce à ce décalage angulaire, lorsque l'on pulvérise un jet de liquide par chaque ouverture d'évacuation 8, ce jet a tendance à se propager à la surface de la paroi interne du conduit auditif externe 2, en direction de la membrane tympanique 3b, selon un trajet sensiblement hélicoïdal. Ainsi avec seulement trois jets de pulvérisation issus de ces ouvertures d'évacuation 8, il est possible d'amener le liquide en contact avec la plus grande partie de la surface de la paroi interne du conduit auditif externe 2, et par là même d'obtenir un nettoyage efficace de ce conduit auditif.

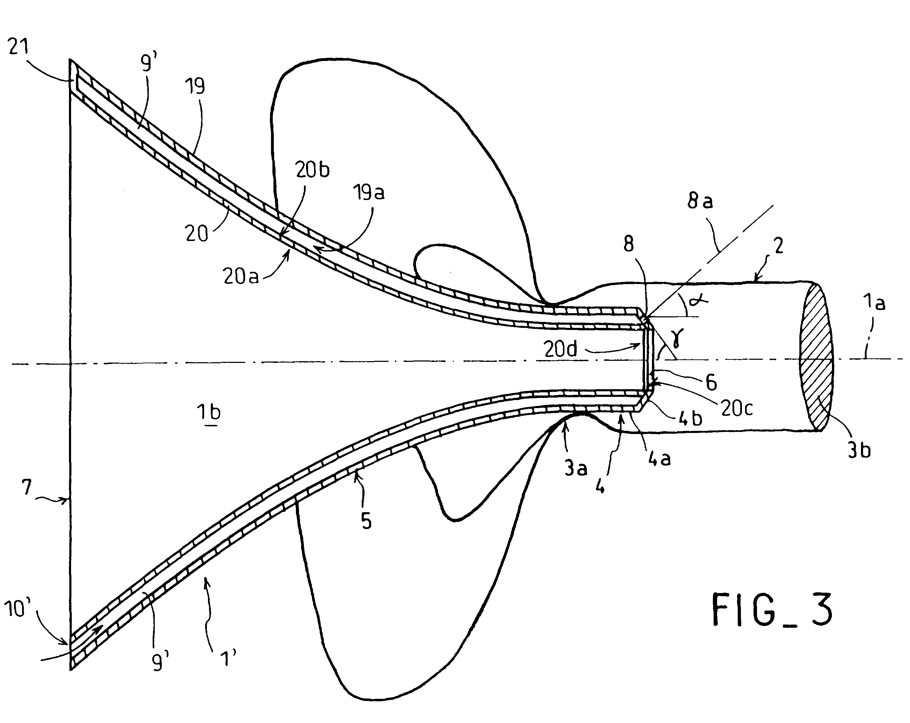

On a représenté sur la figure 3 une autre variante de réalisation d'un embout 1' de l'invention, qui se différencie de l'embout 1 des figure 1 et 2 uniquement par sont conduit d'amenée 9' d'un liquide jusque chaque orifice d'évacuation. Par soucis de simplification, on a conservé les même références pour les parties communes aux embouts 1 et 1'. L'embout 1 ' à la différence de l'embout 1 est constitué de deux éléments concentriques 19 et 20, espacés l'un de l'autre par un espace annulaire 9'. L'élément extérieur 19 est similaire au cornet formant l'embout 1 des figures 1 et 2 , si ce n'est qu'il n'est plus équipé de tuyaux flexibles 9, et ne présente donc plus d'orifice 10 pour le passage de ces tuyaux. L'élément intérieur 20 a la forme d'un cornet de plus petite section que l'élément extérieur 20. La face externe 20b de l'élément intérieur 20 délimite avec la face interne 19a de l'élément extérieur 19 l'espace annulaire 9', et la face interne 20a de l'élément intérieur 20 délimite la cavité intérieure 1b. Les ouvertures d'évacuation 8 de la tête de pulvérisation sont pratiquées dans l'élément extérieur 19, et communiquent avec l'espace annulaire 9'. L'espace annulaire 9' est fermé par une paroi annulaire 21 au niveau de la partie de l'embout 1' qui ne peut pas être introduite dans le conduit auditif, laquelle paroi annulaire est percé d'un orifice 10' pour l'introduction d'un liquide dans l'espace annulaire 9'. En injectant un liquide sous pression dans l'espace annulaire 9' par l'ouverture 10', on alimente chaque orifice d'évacuation 8, l'espace annulaire 9' faisant ainsi office de canal d'amenée du liquide. Au niveau de l'extrémité de pulvérisation de l'embout 1', l'extrémité correspondante 20c de l'élément intérieur 20 vient en contact étanche avec la partie tronconique 4b de l'élément extérieur 19, de telle sorte qu'un liquide injecté dans l'espace annulaire 9' ne puisse pas être évacué par l'ouverture 6 de l'élément extérieur 19. En outre, au niveau de cette extrémité 20c, l'élément intérieur 20 est pourvu d'une ouverture 20d qui communique avec l'ouverture 6, de telle sorte que l'air présent dans le conduit auditif 2 puisse passer dans la cavité intérieure 1b.

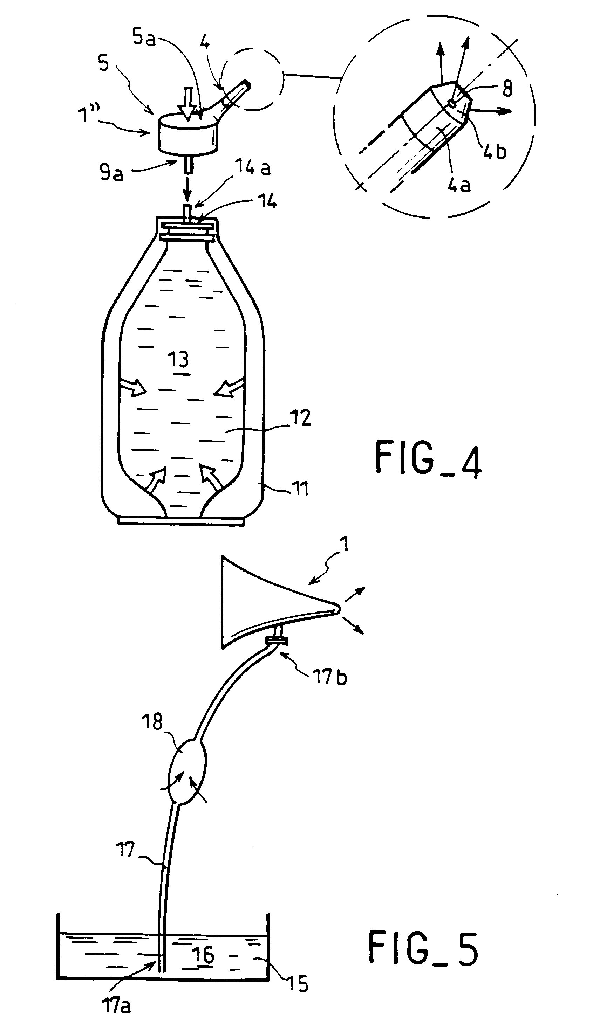

On a représenté à la figure 4, une autre variante possible de réalisation d'un embout 1" de l'invention, qui est destiné à être monté sur un flacon hermétique 11. Un tel flacon est similaire à celui utilisé en rhinologie, pour la pulvérisation d'un liquide de nettoyage des fosses nasales, et ne sera donc pas décrit en détail. Ce flacon 11 comporte une poche interne 12, à l'intérieur de laquelle a été introduit un liquide 13 sous pression. Cette poche 12 est fermée par un système de clapet anti-retour 14, sur lequel vient s'adapter l'embout 1', par l'intermédiaire de sa partie 5, qui est en l'occurrence de forme cylindrique. La tête de pulvérisation 4 est similaire soit à celle de l'embout 1 des figures 1 et 2, soit à celle de l'embout 1' de la figure 3. Dans le premier cas, Les canaux d'amenée 9 du liquide à destination des orifices d'évacuation 8 communiquent avec un unique canal de collecte 9a, qui vient s'adapter sur la sortie 14a de la poche 12. Dans le second cas, le canal de collecte 9a communique directement avec l'espace annulaire 9' de la tête de pulvérisation. Lorsqu'on exerce une pression sur la face supérieure 5a de cette partie 5, on ouvre le système de clapet anti-retour 14 et on libère le liquide 13 contenu dans la poche 12 à destination du canal 9a et par là-même des orifices d'évacuation 8 de la tête de pulvérisation 4 de l'embout 1".

Le dispositif de pulvérisation de la figure 4 est destiné à être utilisé par toute personne non spécialisée dans le domaine O.R.L., contrairement au dispositif de pulvérisation de la figure 5 qui va à présent être décrit. Ce dispositif est constitué d'un récipient 15 contenant un liquide 16, et d'un conduit 17 dont une première extrémité 17a plonge dans le liquide 16, dont la seconde extrémité 17b est reliée aux canaux d'amenée du liquide d'un embout de pulvérisation 1, et qui comporte une poire 18, pour le pompage manuel de liquide à destination de l'embout de pulvérisation 1. Ce dispositif de pulvérisation est plutôt destiné à être utilisé par des praticiens O.R.L., car il permet simultanément à la pulvérisation, une inspection visuelle du conduit auditif externe et de la membrane tympanique. Ce dispositif de pulvérisation pourra également être avantageusement utilisé en chirurgie. Bien entendu, dans le dispositif de la figure 5 il est possible de remplacer l'embout 1 par l'embout 1' de la figure 3. Dans ce cas, le conduit 17 sera relié à l'espace annulaire 9' de l'embout 1'.

Dans les deux exemples de dispositifs de pulvérisation des figures 4 et 5, on pourra utiliser tout type de liquide susceptible de débarrasser le conduit auditif externe de son cérumen, ou de toute autre substance similaire. Ce liquide pourra par exemple être de l'eau stérilisée. De préférence, il s'agira d'un soluté stérile physiologique d'eau de mer, tel que celui actuellement utilisé en rhinologie pour le nettoyage des fosses nasales.

## Revendications

1. Embout (1;1';1") de pulvérisation d'un liquide à l'intérieur du conduit auditif externe (2), du type comportant une tête de pulvérisation oblongue (4), percée d'au moins un orifice d'évacuation (8) qui communique avec un canal (9,9') d'amenée du liquide, et dont l'axe de sortie diverge par rapport à l'axe longitudinal (1a) de la tête de pulvérisation en étant orienté en direction de l'extrémité de ladite tête, de telle sorte qu'une fois la tête de pulvérisation (4) introduite à l'intérieur du conduit auditif (2), le liquide pulvérisé par l'orifice d'évacuation (8) se trouve projeté directement contre la paroi interne (2a) du conduit auditif, caractérisé en ce qu'il comprend une cavité intérieure (1b) qui est distincte du canal (9, 9') d'amenée du liquide et qui présente au moins une première ouverture (6) au niveau de la tête de pulvérisation (4) et une deuxième ouverture (7)au niveau de la partie (5) de l'embout de pulvérisation opposée à la tête de pulvérisation, de telle sorte qu'une fois la tête (4) de l'embout de pulvérisation introduite dans le conduit auditif (2), la partie (5) de l'embout pourvue de la deuxième ouverture (7) est située à l'extérieur de l'oreille, et l'air initialement présent entre l'extrémité de la tête (4)de l'embout de pulvérisation et la membrane tympanique (3b) peut s'évacuer librement vers l'extérieur de l'oreille par la cavité intérieure (1b).

2. Embout de pulvérisation selon la revendication 1 caractérisé en ce que la partie (5) de l'embout de pulvérisation comportant la deuxième ouverture (7) présente une dimension suffisamment importante pour empêcher son passage dans le méat auditif (3a).

3. Embout de pulvérisation selon la revendication 1 caractérisé en ce que les première et deuxième ouvertures (6,7) sont alignées l'une par rapport à l'autre dans l'axe longitudinal (1a) de l'embout, en sorte de permettre l'inspection visuelle du conduit auditif externe (2).

4. Embout de pulvérisation selon les revendications 2 et 3 caractérisé en ce qu'il a la forme d'un spéculum.

5. Embout de pulvérisation selon la revendications 1 caractérisé en ce qu'il comporte deux éléments concentriques (19 , 20), l'élément intérieur (20) délimitant la cavité intérieure (1b), et l'élément extérieur (19) délimitant avec l'élément intérieur (20) un espace annulaire (9'), et en ce que le ou les orifices d'évacuation (8) sont pratiqués dans l'élément extérieur (19) et communiquent avec l'espace annulaire (9'), qui fait office de canal d'amenée du liquide.

6. Embout de pulvérisation selon la revendication 1 caractérisé en ce que la composante longitudinale (8a) de l'axe de sortie de chaque orifice d'évacuation (8) fait, avec l'axe longitudinal (1a) de la tête de pulvérisation (4), un angle α strictement compris entre 30° et 90°, et de préférence égal à 60°.

7. Embout selon la revendication 6 caractérisé en ce que la composante transversale (8b) de l'axe de sortie de chaque orifice d'évacuation (8) fait, avec l'axe longitudinal (1a) de la tête de pulvérisation (4), un angle β strictement compris entre 0° et 90°, et de préférence égal à 45°.

8. Embout selon la revendication 7 caractérisé en ce qu'il comprend au moins trois orifices d'évacuation (8), qui sont régulièrement répartis sur toute la périphérie de la tête de pulvérisation (4), et dont les angles β sont égaux.

9. Dispositif de pulvérisation comportant un récipient (11) qui est rempli avec un liquide (13) sous pression, qui est fermé par un clapet anti-retour (14), et sur lequel est monté embout de pulvérisation visé à l'une quelconque des revendications 1 à 8, dont le ou les canaux d'amenée (9,9') du liquide communiquent avec le récipient et qui permet d'actionner le clapet anti-retour (14) pour libérer le liquide (13) contenu dans le récipient à destination du ou des orifices d'évacuation de l'embout.

10. Dispositif de pulvérisation comportant un embout qui est visé à l'une des revendications 1 à 8, et dont le ou les canaux d'amenée (9, 9') du liquide communiquent avec un récipient ouvert (15), par l'intermédiaire d'un conduit (17), lequel conduit est équipé d'une poire (19) permettant le pompage manuel d'un liquide (16) contenu dans le récipient (15).

11. Dispositif selon les revendications 9 ou 10 caractérisé en ce que le liquide (13,16) est un soluté stérile physiologique d'eau de mer.

## Patentansprüche

1. Aufsatz (1, 1'; 1") zum Zerstäuben einer Flüssigkeit innerhalb des äußeren Gehörgangs (2), von der Art, die einen länglichen Zerstäuberkopf (4) aufweist, der mindestens ein Austrittsloch (8) besitzt, das mit einem Flüssigkeitszufuhrkanal (9, 9') in Verbindung steht und dessen Ausgangsachse in Bezug auf die Längsachse (1a) des Zerstäuberkopfes divergiert, indem sie in Richtung des Endes des Kopfes so ausgerichtet ist, daß, wenn der Zerstäuberkopf (4) ins Innere des Gehörgangs (2) eingeführt ist, die durch das Austrittsloch (8) zerstäubte Flüssigkeit direkt gegen die Innenwand (2a) des Gehörgangs geschleudert wird, dadurch gekennzeichnet, daß er einen inneren Hohlraum (1b) aufweist, der sich vom Flüssigkeitszufuhrkanal (9, 9') unterscheidet und mindestens eine erste Öffnung (6) in Höhe des Zerstäuberkopfes (4) und eine zweite Öffnung (7) in Höhe des dem Zerstäuberkopf entgegengesetzten Bereichs (5) des Zerstäuberaufsatzes aufweist, so daß, wenn der Kopf (4) des Zerstäuberaufsatzes in den Gehörgang (2) eingeführt ist, der Bereich (5) des Aufsatzes mit der zweiten Öffnung (7) sich außerhalb des Ohrs befindet, und die ursprünglich zwischen dem Ende des Kopfes (4) des Zerstäuberaufsatzes und dem Trommelfell (3b) vorhandene Luft durch den inneren Hohlraum (1b) frei nach außerhalb des Ohres entweichen kann.

2. Zerstäuberaufsatz nach Anspruch 1, dadurch gekennzeichnet, daß der die zweite Öffnung (7) aufweisende Bereich (5) des Zerstäuberaufsatzes groß genug ist, um sein Eindringen in den Gehörgang (3a) zu verhindern.

3. Zerstäuberaufsatz nach Anspruch 1, dadurch gekennzeichnet, daß die erste und die zweite Öffnung (6, 7) zueinander fluchtend in der Längsachse (1a) des Aufsatzes ausgerichtet sind, um die visuelle Untersuchung des äußeren Gehörgangs (2) zu ermöglichen.

4. Zerstäuberaufsatz nach den Ansprüchen 2 und 3, dadurch gekennzeichnet, daß er die Form eines Spekulums hat.

5. Zerstäuberaufsatz nach Anspruch 1, dadurch gekennzeichnet, daß er zwei konzentrische Elemente (19, 20) aufweist, wobei das innere Element (20) den inneren Hohlraum (1b) und das äußere Element (19) mit dem inneren Element (20) einen ringförmigen Raum (9') begrenzt, und daß das Austrittsloch oder die Austrittslöcher (8) im äußeren Element (19) ausgebildet ist (sind) und mit dem ringförmigen Raum (9') in Verbindung steht (stehen), der als Flüssigkeitszufuhrkanal dient.

6. Zerstäuberaufsatz nach Anspruch 1, dadurch gekennzeichnet, daß die Längskomponente (8a) der Ausgangsachse jedes Austrittslochs (8) mit der Längsachse (1a) des Zerstäuberkopfes (4) einen Winkel α bildet, der strikt zwischen 30 und 90° liegt und vorzugsweise 60° beträgt.

7. Aufsatz nach Anspruch 6, dadurch gekennzeichnet, daß die Querkomponente (8b) der Ausgangsachse jedes Austrittslochs (8) mit der Längsachse (1a) des Zerstäuberkopfes (4) einen Winkel β bildet, der strikt zwischen 0° und 90° liegt und vorzugsweise 45° beträgt.

8. Aufsatz nach Anspruch 7, dadurch gekennzeichnet, daß er mindestens drei Austrittslöcher (8) aufweist, die regelmäßig über den ganzen Umfang des Zerstäuberkopfes (4) verteilt sind und deren Winkel β gleich sind.

9. Zerstäubervorrichtung mit einem Behälter (11), der mit einer unter Druck stehenden Flüssigkeit (13) gefüllt ist, der von einem Rückschlagventil (14) verschlossen wird und auf den der Zerstäuberaufsatz gemäß einem beliebigen der Ansprüche 1 bis 8 montiert ist, dessen Flüssigkeitszufuhrkanal oder -kanäle (9, 9') mit dem Behälter in Verbindung steht (stehen) und der es ermöglicht, das Rückschlagventil (14) zu betätigen, um die im Behälter enthaltene Flüssigkeit (13) in Richtung des Austrittslochs (der Austrittslöcher) des Aufsatzes freizugeben.

10. Zerstäubervorrichtung mit einem Aufsatz gemäß einem der Ansprüche 1 bis 8, dessen Flüssigkeitszufuhrkanal oder -kanäle (9, 9') mit einem offenen Behälter (15) über eine Leitung (17) in Verbindung steht (stehen), wobei die Leitung mit einem Ballon (18) ausgestattet ist, der das manuelle Pumpen einer im Behälter (15) enthaltenen Flüssigkeit erlaubt.

11. Vorrichtung nach den Ansprüchen 9 oder 10, dadurch gekennzeichnet, daß die Flüssigkeit (13, 16) eine sterile physiologische Meersalzlösung ist.

## Claims

1. A nozzle (1; 1'; 1") for spraying a liquid into the external auditory duct (2), of the type which has an oblong spray head (4) in which there is at least one discharge port (8) communicating with a liquid intake channel (9, 9'), the outlet axis of which diverges from the longitudinal axis (1a) of the spray head by being turned in the direction of the end of the aforesaid head in such a way that once the spray head (4) is inserted inside the auditory duct (2), the liquid sprayed through the discharge port (8) goes direct against the inner wall (2a) of the auditory duct, characterised by the fact that it has an internal cavity (1b) which is distinct from the liquid intake channel (9, 9') and has at least a first opening (6) at the spray head (4) and a second opening (7) at the portion (5) of the spray nozzle opposite the spray head, so that once the head (4) of the spray nozzle is inserted into the auditory duct (2), the portion (5) of the nozzle in which there is the second opening (7) is located outside the ear, and the air initially present between the end of the head (4) of the spray nozzle and the car drum (3b) can be freely discharged from the ear through the inner cavity (1b).

2. A spray nozzle according to Claim 1, characterised by the fact that the portion (5) of the spray nozzle which has the second opening (7) is large enough to prevent it from passing into the auditory meatus (3a).

3. A spray nozzle according to Claim 2, characterised by the fact that the first and second openings (6, 7) are aligned with each other in the longitudinal axis (1a) of the nozzle to allow visual inspection of the external auditory duct (2).

4. A spray nozzle according to Claims 2 and 3, characterised by the fact that it is speculum shaped.

5. A spray nozzle according to Claim 1, characterised by the fact that it has two concentric parts (19, 20), the inner part (20) delineating the inner cavity (1b), and the outer part (19) with the inner part (20) delineating an annular space (9'), and by the fact that the discharge port or ports (8) are made in the outer part (19) and are in communication with the annular space (9') which acts as the liquid intake channel.

6. A spray nozzle according to Claim 1, characterised by the fact that the longitudinal component (8a) of the outlet axis of each discharge port (8) and the longitudinal axis (1a) of the spray head (4) form an angle α which is strictly between 30° and 90°, and preferably equal to 60°.

7. A nozzle according to Claim 6, characterised by the fact that the transverse component (8b) of the outlet axis of each discharge port (8) and the longitudinal axis (1a) of the spray head (4) form an angle β which is strictly between 0° and 90°, and preferably equal to 45°.

8. A nozzle according to Claim 7, characterised by the fact that it has at least three discharge ports (8) which are regularly spaced round the whole periphery of the spray head (4), the β angles of which are equal.

9. A spraying device with a receptacle (11) which is filled with a pressurised liquid (13) and is closed with a non return valve (14), and to which is fitted the spray nozzle mentioned in any of the Claims 1 to 8, the liquid intake channels (9,9') of which are in communication with the receptacle and allow the non return valve (14) to operate to release the liquid (13) contained in the receptacle towards the discharge port or ports of the nozzle.

10. A spraying device with a nozzle which is mentioned in any of the Claims 1 to 8, the liquid intake channels (9, 9') of which are in communication with an open receptacle (15) by means of a line (17), this line being fitted with a squeeze bulb (18) which enables a liquid (16) contained in the receptacle (15) to be pumped by hand.

11. A device according to Claims 9 or 10, characterised by the fact that the liquid (13, 16) is a sterile physiological sea water solution.
